# EUROPEAN PATENT SPECIFICATION

(11) **EP 2 739 338 B1**
(45) Date of publication and mention of the grant of the patent: **17.05.2017**
(21) Application number: 12761670.4
(22) Date of filing: 02.08.2012
(51) Int. Cl.: A61M 25/02, A61M 5/158

(54) **PROTECTION AND ACCOMMODATION SYSTEM, IN PARTICULAR FOR A DEVICE FOR DELIVERING A TREATMENT TO A PATIENT**
SCHUTZ UND AUFNAHMEVORRICHTUNG, INSBESONDERE FUER EINE VORRICHTUNG ZUR ABGABE EINER SUBSTANZ AN EINEN PATIENTEN
SYSTEME DE PROTECTION ET RECEPTION, EN PARTICULIER POUR UN DISPOSITIF POUR DELIVRER UNE SUBSTANCE A UN PATIENT

(30) Priority: 04.08.2011 IT TO20110729
(43) Date of publication of application: 11.06.2014
(73) Proprietor: Gugliotta, Filippo, 10128 Torino (IT)
(72) Inventor: Gugliotta, Filippo, 10128 Torino (IT)
(74) Representative: Robba, Pierpaolo
(86) International application number: PCT/IB2012/053957
(87) International publication number: WO 2013/041985

(56) References cited:
- WO-A1-95/33508
- WO-A1-2006/120623
- US-A- 4 896 465
- US-A- 5 193 574
- US-A- 5 226 892
- US-A1- 2006 089 600
- US-A1- 2008 097 334
- US-A1- 2009 143 741
- US-A1- 2011 054 409

## Description

### Technical Field

The present invention refers to a protection and accommodation system, in particular for a device for delivering a treatment to a patient. More particularly, the invention refers to a protection and accommodation system that can be used for instance in the field of the delivery of treatments of a mechanical type, for example by means of counter-pulsers, of an electrical type, for example by means of electrodes, of a pharmacological type, for example by means of catheters, by intravenous, intra-arterial, subcutaneous, intramuscular way.
Reference will be made in the following, by way of example, specifically to the field of intravenous drug administration. This field of application is not to be considered as limitative, the use of the system being possible for the other kinds of treatment referred to above as well as for further ones.

### Prior Art

It is known in the field to employ protection and/or accommodation systems for devices for administering drugs to patients by intravenous way, for instance by means of catheters.
US 4 896 465 A discloses a protection and accommodation system for a device for delivering a treatment to a patient wherein said system comprises a support structure arranged to be attached in a removable manner to the patient's body, and a retaining structure associated with said support structure and comprising a resilient deformable material like sponge for clamping the cannula of the delivering device.
WO 2006/120623 A1 discloses a protection and accommodation system for a device for delivering a treatment to a patient comprising a support structure arranged to be attached in a removable manner to the patient's body, and a retaining structure associated with said support structure and comprising a support part made of foamed deformable material having oppositely facing jaws for, in between them, taking hold of a section of a tube of the delivering device.
US 2011/054409 A1 discloses a protection and accommodation system for a device for delivering a treatment to a patient comprising a support structure arranged to be attached in a removable manner to the patient's body, and a retaining structure associated with said support structure (110) and comprising a resilient retainer secured for securing a medical article.
US 5 193 574 A discloses a protective structure for connection site for medical use comprising a housing divided in two half shells having raised adges for retaining delivering tubes within a foam provided therein.

However, known systems are affected by several drawbacks.

### Summary of the Invention

It is an object of the present invention to realize a system of the above-mentioned type that is improved and at the same time can be manufactured in an easy and cost-effective way.

According to the present invention, this and other objects are attained by a system of the above-identified type and realized according to the appended Claim 1.

### Brief Description of the Drawings

Further features and advantages of the present invention will become apparent from the following description, which is given by way of non-limiting example only, with reference to the attached drawings, wherein:
- Figures 1 to 4 are plan views of a first exemplary embodiment of a protection and accommodation system that is not an embodiment of the present invention;
- Figures 5 and 6 are plan views corresponding to Figures 3 and 4, but showing a second exemplary embodiment of a system according to the present invention;
- Figures 7 and 8 are perspective views of preferred constructive details of the second embodiment of the system;
- Figure 9 is a perspective view of a third embodiment according to the present invention;
- Figure 10 is a perspective view of a fourth exemplary embodiment of a system according to the present invention;
- Figure 11 is a perspective view of a fifth embodiment of a protection and accommodation system that is not an embodiment of the present invention.

### Detailed Description of the Invention

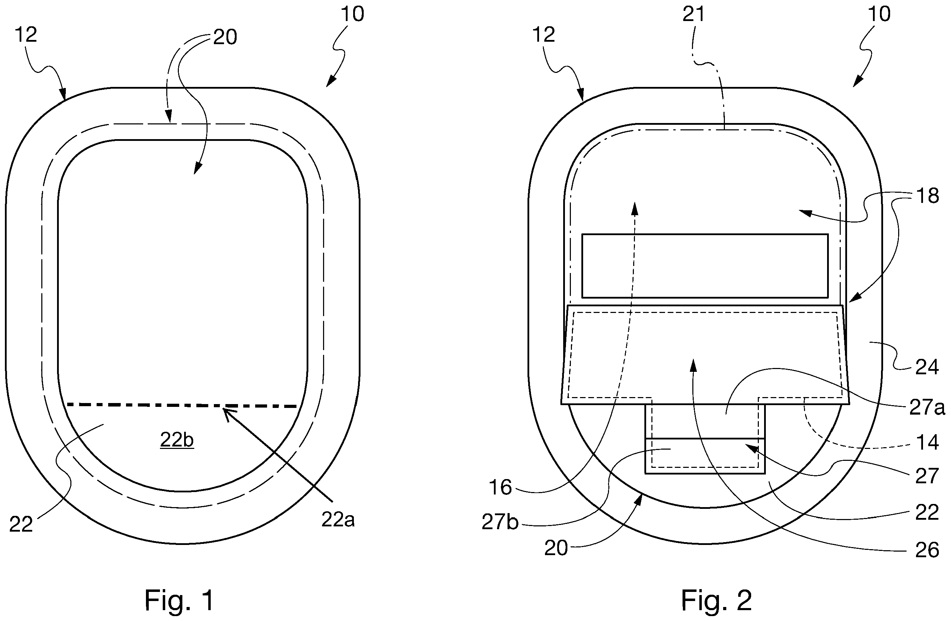

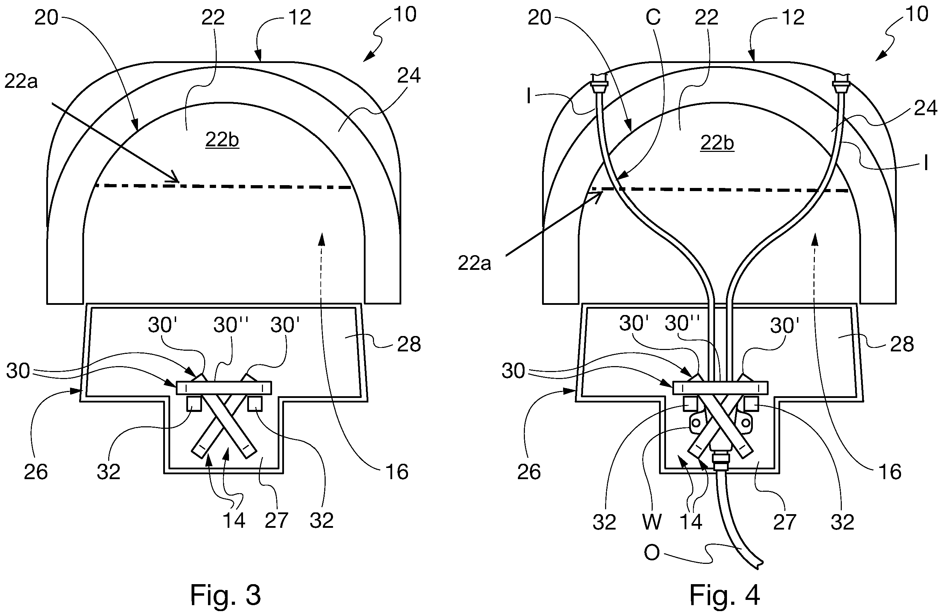

With reference to Figures 1 to 4, there is indicated with 10 a first embodiment of a protection and accommodation system that is not an embodiment of the present invention. The system 10 is suitable especially for the use of a delivery device C (see Figure 4) for administering drugs to a patient by intravenous way. For example, the delivery device is a catheter C. As can be seen in Figure 4, the catheter has an arrangement known per se including preferably a pair of inlet cannulas I, an outlet cannula O and an intermediate group of lugs W interposed between inlet and outlet cannulas I and O. As known to a person skilled in the art, the intermediate group of lugs W has the function of facilitating grip and handling of catheter C.

With reference to Figures 1, 3 and 4, the system 10 is shown from the viewpoint of an observer watching the system 10 from the outside when it is applied to the patient's body.
Figure 2, instead, shows the system 10 as this could been seen by an observer who could watch the system 10 from the inside to the outside when it is applied to the patient's body. In view of the above, in the present description and in the appended claims the adjective "inner" and the terms deriving therefrom are to be intended, in the use of system 10, as referring to a direction towards - or to a position relatively closer to - the patient's body. On the contrary, as far as the adjective "outer" and the terms deriving therefrom are concerned, they are to be intended, in the use of system 10, as referring to a direction away from - or to a position relatively farther from - the patient's body.

The system 10 comprises a support structure 12 arranged to be attached in a removable manner to the patient's body (for instance by means of lacing, bandaging or self-adhesive means) and a retaining structure 14 (better visible in Figure 3) associated with said support structure 12 and arranged to be coupled with at least one fixing portion of a delivery device C. As described above, in the embodiments of the present invention, the delivery device is preferably a catheter C, wherein the fixing portion consists, by way of example but not necessarily, by the intermediate group of lugs W.

In the first embodiment, the support structure 12 defines in its inside a cavity 16 (see Figures 2 to 4) arranged to accommodate at least one portion of catheter C, for instance the inlet cannulas I and/or the outlet cannula O. Optionally, the support structure 12 in use is arranged for assuming a cover arrangement (shown in Figures 1 and 2), wherein it is superposed to - and covers - the retaining structure 14, and a release arrangement (shown in Figures 3 and 4), wherein it allows to reach the retaining structure 14.

Still in this embodiment, in use the support structure 12 includes a pocket that forms the cavity 16. Preferably, the support structure 12 is of a multi-layer type. For example, it includes a first inner sheet 18 and a second outer sheet 20 mutually overlapping and connected to each other so as to form the pocket-like portion located in the space between them. Advantageously the inner sheet 18 and the outer sheet 20 are made of a flexible material, e.g. based on polyethylene, polyester, polyamide, nylon fibers, preferably charged with silver nitrates for their anti-bacterial and anti-inflammatory properties. By way of example, the inner sheet 18 and the outer sheet 20 can be connected to each other for a segment 21 (indicated with a dashed-dotted line in Figure 2) of their respective peripheral portions overlapped by sewing, gluing, welding or other methods known to a person skilled in the art.

Preferably, the support structure 12 includes a cover flap 22 which is oscillatable so as to cover the retaining structure 14 when the support structure 12 is in the cover arrangement. The cover flap is further oscillatable so as to allow to reach said retaining structure 14 when the multi-layer support structure is in the release arrangement. With reference to Figure 1, it can be seen how the cover flap 22 is superposed on a patient's body part to be protected. With reference to Figures 3 and 4, instead, it can be seen that, with respect to what is shown in Figure 1, the cover flap 22 is folded against the remaining of the supporting structure 12 (e.g. against the outer face of the outer sheet 20), while uncovering the part formerly covered of the patient's body and the retaining structure 14.

More preferably, the cover flap 22 can be fixed in a removable manner to the patient's body when the multi-layer support structure 12 is in the cover arrangement, for instance, the cover flap 22 is provided with a self-adhesive portion 24 on its inner face. In this embodiment, the cover flap 22 is defined by a portion of the outer sheet 20 projecting laterally beyond the inner sheet 18 so as to be able to cover the retaining structure 14. For instance, the self-adhesive portion 24 is located at the periphery of the inner face of the outer sheet 20.

According to the invention, advantageously, the cover flap 22 can be realized opaque, or transparent, or further provided with an opaque portion and a transparent portion. With reference to the last-mentioned hypothesis, in the annexed figures the boundary between the opaque portion and the transparent one is indicatively represented by a dashed-dotted line 22a and the transparent portion is indicatively represented by the reference numeral 22b. An at least partially transparent cover flap 22 allows in particular to solve the problem of how to observe the health conditions of the area where the outlet cannula O penetrates the patient's body, without removing the cover flap 22. Said area of penetration of the outlet cannula O into the patient's body is indeed known to be exposed to the risk of infections.

Preferably, the multi-layer support structure 12 further comprises a bearing flap 26 on which the retaining structure 14 is supported and which is arranged to be covered by the cover flap 22 when the multi-layer support structure 12 is in the cover arrangement. More preferably, the retaining structure 14 is held on the outer face of the bearing flap 26. In this embodiment, the cover flap 26 is located in vicinity of the opening of the cavity 16, defined for instance by the pocket formed by the inner sheet 18 and the outer sheet 20. Optionally the bearing flap 26 is defined by a portion of the inner sheet 18 projecting laterally beyond said cavity 16. In a preferred way, the bearing flap 26 includes a tab or gripping tongue 27 in order to facilitate gripping of said flap during the step of coupling the delivery device C to the retaining structure 14.

More preferably, the inner face of the gripping tongue 27, which is located opposite to the retaining structure 14, has a proximal portion 27a, relatively closer to the remaining of the bearing flap 26 and provided with an adhesive, and a distal portion 27b, relatively farther from the remaining of the bearing flap 26 and free of adhesive. The proximal portion 27a is advantageously provided with a covering film (not shown).

In the illustrated embodiment, the retaining structure 14 essentially lies on the gripping tongue 27 at its outer face.

Preferably, the retaining structure includes releasable coupling means 14 arranged to lock the fixing portion W. More preferably, the releasable coupling means 14 are of the type having micro-hooks and micro-loops (the so-called "hook-and-loop fastener"), for instance a fastener commonly known under the trade name Velcro®, and are arranged to embrace said fixing portion W. Still more preferably, the hook-and-loop fastener 14 includes a retaining surface 28 mounted on the support structure 12, and one or more holding straps 30 (three straps are illustrated in the embodiment) able to be releasably coupled to said retaining surface 28 so as to embrace and lock in position the fixing portion W. For example, the retaining surface 28 comprises a plurality of micro-loops, while the holding strap (or each of the holding straps) 30 comprises a plurality of micro-hooks able to be coupled to the micro-loops provided on the retaining surface 28. A person skilled in the art may also conceive to reverse the mutual arrangement of micro-loops and micro-hooks between the retaining surface 28 and the holding straps 30. In this embodiment the holding straps are in the form of bands or stripes, but they can be replaced with elements having a different form, e.g. with arrangements having a plurality of arms (by way of example, with a cross layout) arranged for surmounting in different positions the fixing portion W in order to ensure fixed locking of the delivery device, for instance of the catheter C. Advantageously, though not necessarily, the holding straps 30 have the micro-hooks (or possibly the micro-loops) along their entire face arranged for being coupled to the retaining surface 28. Alternatively, the holding straps 30 can be provided with the micro-hooks (or possibly micro-loops) only at their distal ends.

In the illustrated embodiment the holding straps 30 include a pair of holding straps 30' arranged for surmounting the fixing portion W by forming a cross when observed in plan view. Furthermore, the holding straps 30 preferably included a safety holding strap 30" arranged to surmount the fixing portion W and the holding straps 30' at the ends of the latter.

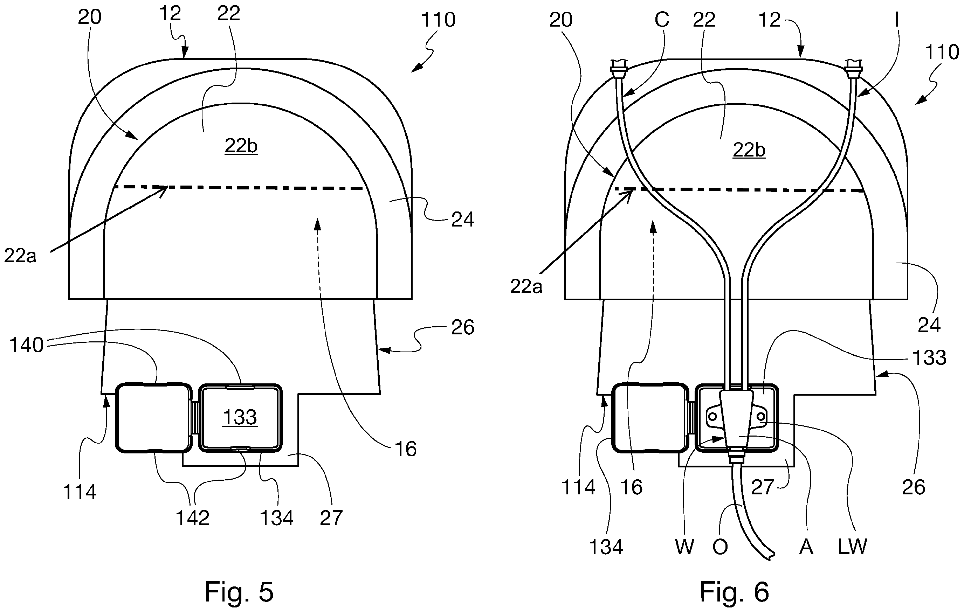

Preferably, the retaining structure 14 can include stop means 32 arranged to abut against the fixing portion W. In the embodiment illustrated in Figure 4, the stop means include a pair of stop projections 32 which are preferably made of soft material and against which the fixing portion, for instance the intermediate group of lugs W, abuts. Advantageously, the stop projections 32 are arranged to hold and support a pair of side lugs LW (better visible in Figure 6) of the intermediate group of lugs W and define between them a gap (not numbered) into which a terminal appendix A (better visible in Figure 6) of the intermediate group of lugs W can be inserted. Preferably, the stop means 32 are located on the retaining portions 28 of the releasable coupling means 14.

Preferably, the intermediate group of lugs W is arranged for being located on the retaining structure 14 in such a way that the stop means 32 prevent the intermediate group of lugs W from moving away from the patient's body. More preferably, the intermediate group of lugs W is located in such a way that the stop means 32 face that portion of the side lugs LW which is directed towards the inlet cannula(s) I. In this way the side lugs LW abut against the stop means 32 with locking action when the intermediate group of lugs is pull-stressed undesirably in a direction away from the patient's body.

A method of installing or applying the first embodiment of the system 10 according to the present invention will be now described in brief.

The application method starts with a step of inserting the device for delivering treatments to the patient's body. In this step an operator inserts the catheter C (or the generic device for delivering treatments) in a patient's body region suitable for delivering the treatment. In the illustrated embodiment the operator inserts the needle (not shown) of the catheter C into a patient's vein.

After that, the application method continues with a step of positioning the system 10 with respect to the patient's body. In this step, for convenience, the operator simply applies the fixing portion W by resting it onto the retaining structure 14, without coupling or steadily connecting the fixing portion W to the system 10 yet. In this step the supporting structure 12 is in the release arrangement, thus allowing the operator to reach freely the retaining structure 14.

Preferably, in this step, at first the operator puts the fixing portion W onto the stop means 32. For instance, the operator puts the intermediate group of lugs W of catheter C between the pair of stop projections 32, with a slight interference between them and leaving the pair of side lugs LW in contact against said projections 32. At the end of the application method, the stop projections 32 prevent the catheter from withdrawing and the relating risk that the needle inserted in the patient's body may come out.

Thereafter, still during this step, the operator brings the system 10, on which the fixing portion W rests, away from - or towards - the region for delivering the treatment, trying to reach the optimal distance for that. Preferably, in this step, the operator removes the covering film applied to the proximal portion 27a, thus leaving the adhesive free, and he makes the gripping tongue 27 slide against the patient's body during the positioning operations. In a more preferred way, the gripping tongue 27 slides with its distal portion 27b against the patient's body.

This movement of the system 10 with respect to the region for delivering the treatment is aimed at reaching an optimal distance between the fixing portion W of the catheter C and the region for delivering the treatment, for instance for preventing the outlet cannula O from forming folds or bends that may jeopardize the correct use and functioning of catheter C.

Subsequently, the application method continues with a coupling step in which the fixing portion W is fixedly connected to the system 10. In this step, the operator couples the fixing portion W with the releasable coupling means 14 after reaching the optimal positioning of said fixing portion W with respect to the region for delivering the treatment.

Preferably, in this step, the operator encircles the fixing portion W applied by means of the hook-and-loop-fastener 14. For instance, the operator couples the holding straps 30 to the retaining surface 28 so as to embrace and lock in position the fixing portion W. It is to be noted that the optional use of the bearing flap 26 the operator can exert a pressure on the retaining structure, in this case the releasable coupling means 14, without effecting any direct squeezing on the patient's body. More particularly, the operator can exert a pressure through the inner face and the outer face of the bearing flap 26 in order to determine coupling of the releasable coupling means 14 and the fixing portion, avoiding to touch the patient's body during his action.

After that, the method of use continues with a fixing step, in which the system 10 is fixed onto the patient's body. In this step, the system has already been coupled to the fixing portion W by locking the support structure 12, and the operator couples the system 10 to the patient's body by means of a self-adhesive. Advantageously, the fixing of the support structure 12 is obtained by means of a self-adhesive which detachably allows adherence of the support structure 12 to the patient's body. For instance, in this step, the operator pushes the face of the proximal portion 27a provided with adhesive against the patient's body.

Afterwards, the application method continues with a containment step. In this step, after having fixed on the patient's body the system 10 coupled to the fixing portion W, the operator inserts a part of the delivery device C, e.g. the inlet cannulas I, into the cavity 16. In this way, an optimal accommodation of the delivery device C in the system 10 is obtained.

Finally, the application method ends with a covering step, in which the support structure 12 is brought by the operator from the release arrangement to the cover arrangement, so as to protect the delivery device C. Preferably the cover flap 22 is brought to cover the retaining structure 14 and, advantageously, it is fixed onto the patient's body, for instance by means of a self-adhesive. In the illustrated embodiment, the protection of the delivery device C can be obtained by means of the self-adhesive portion indicated with 24 located peripherally on the cover flap 22.

Evidently, in using the system 10, whenever it is desired to proceed to the delivery of the treatment, it will be possible for an operator to bring the support structure 12 from the cover arrangement to the release arrangement and take the inlet cannulas I out of the cavity 16, so that the drugs to be administered to the patient are allowed to flow through them, the outlet cannula O being already correctly inserted in the patient's body by means of its needle.

At the end of the treatment delivery, the support structure 12 can be brought back to the cover arrangement after having inserted again the outlet cannulas O into the cavity 16.

With reference to Figures 5 to 8, a second embodiment of the system according to the present invention will be now described. Said second embodiment is generally indicated with reference numeral 110.

Features or elements that are similar to those of the first illustrated embodiment, or have an equivalent function, are identified with the same alphanumeric references. For reasons of brevity, the description of these features and elements will not be repeated in the following, but reference will be made to what has been disclosed in the description of the first embodiment. Regarding features and elements that show differences that are essential from the structural and/or functional point of view with respect to the first embodiment, they are identified with the same alphanumeric references increased by 100. Finally, regarding features and elements that are new with respect to the first embodiment, the reference numerals are the continuations of the numbering used for the first embodiment but increased by 100.

As can be appreciated especially in Figures 5 and 6, the system 110 has a retaining structure 114 different with respect to the first embodiment.

One of the differences lies in the fact that the retaining structure 114 includes a deformable, preferably elastomeric material 133, arranged to be deformed by a compression performed by an operator, who pushes the fixing portion W against the elastomeric material 133, and arranged for embedding and retaining the fixing portion W by means of a partial, elastic return performed by the deformable material 133 after the compression performed by the operator. Preferably, the elastomeric material is a polymer 133. More preferably, the polymer 133 is a polymer included in the list consisting of silicone, inert polyurethane; other suitable deformable materials are clay-based materials, waxes, shape-memory materials, or polyester-based materials. For instance, the elastomeric material 133 has also adhesive properties, which facilitates coupling to the fixing portion W.

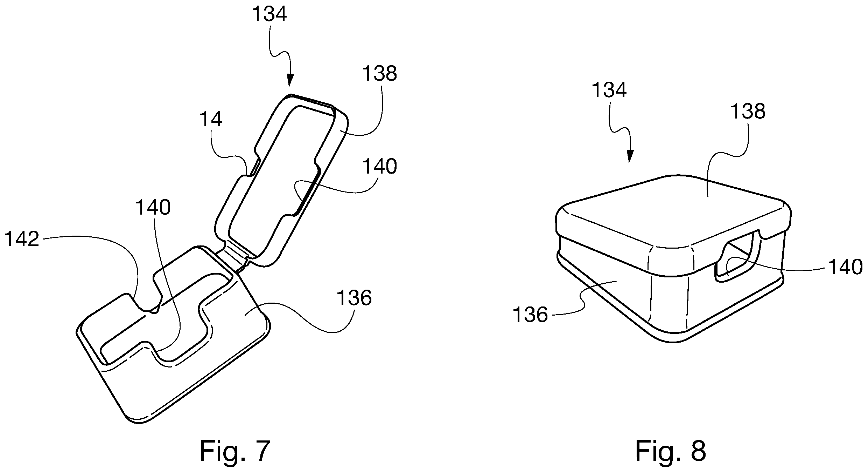

Another difference lies in the fact that the retaining structure 114 includes a casing 134 that receives the elastomeric material 133. An example of the casing 134, shown without the elastomeric material 133 at its inside, is illustrated in greater detail in Figures 7 and 8.

With reference in particular to Figures 7 and 8, the casing 134 preferably includes a tray 136 that receives said elastomeric material 133 at its inside. In a more preferred way, the casing 134 includes a reclosable lid 138 able to be coupled with said tray 136. Advantageously, though not necessarily, the casing 134 has an essentially parallelepiped form.

Preferably, the casing 134 has a (first) passageway 140 arranged to be passed through by a portion (for instance, the inlet cannulas I) of the delivery device C. In the second embodiment the (first) passageway, for instance realized as a (first) recess 140, is provided on the tray 136 and/or on the lid 138, conveniently on the lateral surface of the latter. In a more preferred way, the casing 134 further has a second passageway 142 arranged to the passed through by a portion (for instance, the outlet cannula O) of the delivery device C. In this embodiment the second passageway, for instance made as a second recess 142, is provided on the tray 136 and/or on the lid 138. Advantageously, though not necessarily, the first passageway 140 and the second passageway 142 are made by laterally opposite parts of the casing 134, for instance by the tray 136 and/or by the lid 138. Said passageways can be any in number for the passage of a plurality of catheters, if necessary, and/or of devices having multiple inlets and/or outlets.

Preferably, the elastomeric material 133 - and therefore the possible casing 134 (e.g. including the tray 136) containing the same - is held by the bearing flap 26, advantageously on the outer face of the same. As is clear to a person skilled in the art in the light of the present description, the elastomeric material can also be applied directly to the support structure 12, for instance to the bearing flap 26, without using the casing 134.

The differences discussed above do not affect substantially the functioning of the second embodiment of the system 110. Summarizing as much as possible, instead of using the releasable coupling means 14, the support structure 114 provides for the use of the elastomeric material 133, possibly though not necessarily in combination with the casing 134, in order to retain the fixing portion W.

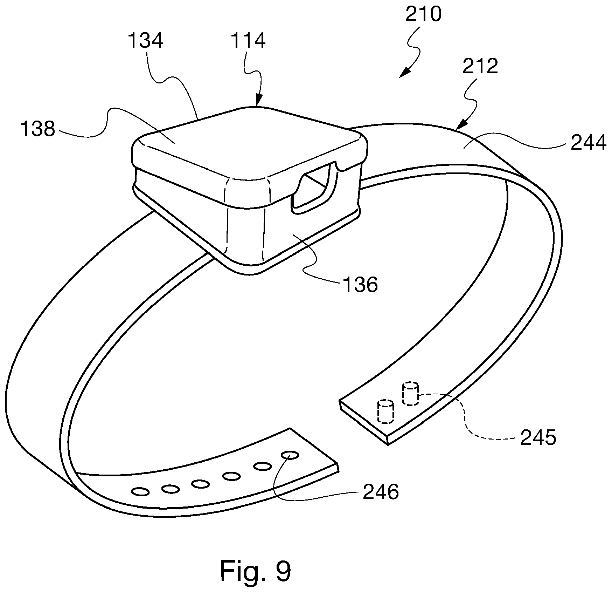

With reference to Figure 9, a third embodiment of the system according to the present invention will now be described. Said third embodiment is generally indicated with reference numeral 210.

Features or elements that are similar to those of the second illustrated embodiment, or have an equivalent function, are identified with the same alphanumeric references. For reasons of brevity, the description of these features and elements will be not repeated in the following, but reference will be made to what has been disclosed in the description of the first and second embodiments. Regarding features and elements that show differences that are essential from the structural and/or functional point of view with respect to the second embodiment, they are identified with the same alphanumeric references increased by 200. Finally, regarding features and elements that are new with respect to the first embodiment, the reference numerals are the continuations of the numbering used for the first embodiment increased by 100.

As can be appreciated, the system 210 has a support structure 212 different with respect to the first and second embodiments.

One of the differences lies in the fact that the support structure 212 includes a band 244 wearable on - or applicable to - the patient's body. For instance, the band is a bracelet or armband 244 that can be laced onto a patient's arm. For instance, the band 244 is made of a flexible material and includes one or more pins 245 located in the proximity of a longitudinal end of the band 244, and a corresponding plurality of through-holes 246 located in the proximity of the opposite longitudinal end of said band 244 and arranged for receiving the pin(s) 245 for coupling. In this way, by positioning the band 244 onto the patient's body and coupling the pin(s) 245 in the desired through-hole(s) 246, a lacing of the band 244 is obtained by securing it comfortably and in a customized way around the patient's body. In the illustrated embodiment, the pins 245 and the through-holes 246 are located on a face of the band 244 which in use is the outer face, away from the patient's body. Moreover, the pins 245 and the through-holes 246 are aligned in a longitudinal direction of the band 244.

Regarding the retaining structure 114, it is essentially identical to the one described in the second embodiment and therefore it will not be described further. As is clear to a person skilled in the art in the light of the present description, alternatively, the retaining structure 14 described in connection with the first embodiment can also be applied to the system 210. For example, in this case the retaining structure 14 could be applied to the outer face of the wearable band 244.

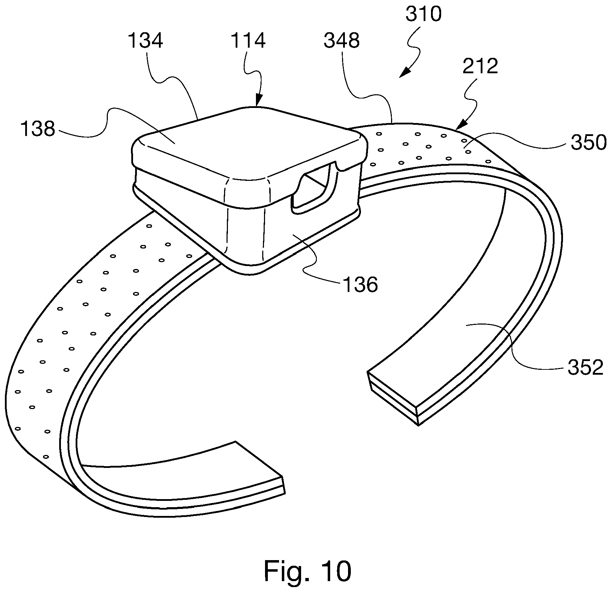

With reference to Figure 10, a fourth embodiment of the system according to the present invention will now be described. Said fourth embodiment is generally indicated with reference numeral 310.

Features or elements that are similar to those of the third illustrated embodiment, or have an equivalent function, are identified with the same alphanumeric references. For reasons of brevity, the description of these features and elements will be not repeated in the following, but reference will be made to what has been disclosed in the description of the preceding embodiments. Finally, regarding features and elements that are new with respect to the second embodiment, the reference numerals are the continuations of the numbering used for the third embodiment increased by 100.

As can be appreciated, the system 310 has a support structure 312 different with respect to the preceding embodiments.

One of the differences lies in the fact that the support structure 312 includes a patch 348 that can be applied onto the patient's body. For instance, the patch 348 includes a support layer 350 operatively directed outwards, arranged to be applied onto the patient's body and covered by a protective element.

Regarding the retaining structure 114, it is essentially identical to the one described in the second and third embodiments and therefore it will not be described further. As is clear to a person skilled in the art in the light of the present description, alternatively, the retaining structure 14 described in connection with the first embodiment can also be applied to the system 310. For example, in this case the retaining structure 14 could be applied to the outer face of the patch 348, advantageously on the outer face of the support layer 350.

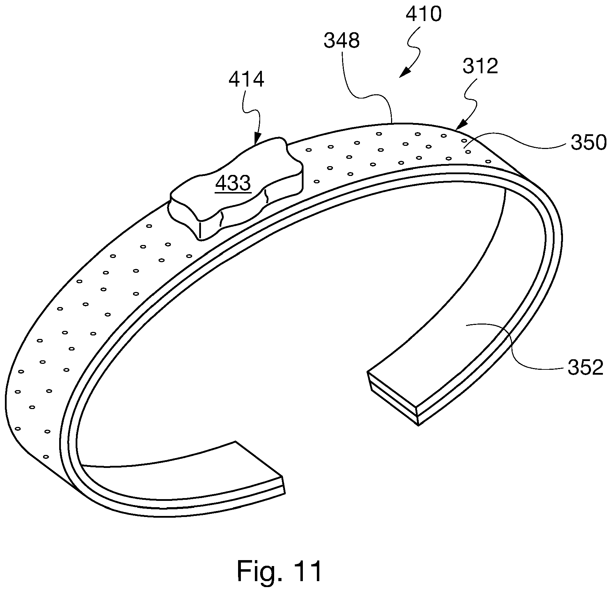

With reference to Figure 11, a fifth embodiment of a protection and accommodation system that is not an embodiment of the present invention will now be described. Said fifth embodiment of the system is generally indicated with reference numeral 410.

Features or elements that are similar to those of the fourth illustrated embodiment, or have an equivalent function, are identified with the same alphanumeric references. For reasons of brevity, the description of these features and elements will be not repeated in the following, but reference will be made to what has been disclosed in the description of the preceding embodiments. Regarding features and elements that show differences that are essential from the structural and/or functional point of view with respect to the third embodiment, they are identified with the same alphanumeric references increased by 300.

As can be appreciated, the system 410 has a support structure 312 essentially identical to the one of the fourth embodiment. Instead, as can be appreciated in Figure 11, the system 310 has a retaining structure 414 different from the one illustrated in the second to fourth embodiments.

One of the differences lies in the fact that the retaining structure 414 includes only the elastomeric material 433, without the presence of the casing 134 indicated in the preceding embodiments. The elastomeric material 433 is essentially identical to the one described with reference to the preceding embodiments and in particular indicated with 133 in the second embodiment. Preferably, the elastomeric material 433 is applied directly (for instance glued) to the outer face of the support structure 312, i.e. to the outer face of the patch 348, for instance to the outer face of the support layer 350.

The technical features that differentiate the various embodiments described and illustrated are freely interchangeable with one another, where compatible.

Obviously, as far as the principle of the invention remains the same, the embodiments and details of the invention can be widely varied with respect to what has been described and shown merely as a non-limiting example, without departing from the scope of the invention as defined in the appended claims.

### References in the Figures

- C: Delivery device or catheter
- I: Inlet cannulas
- O: Outlet cannulas
- W: Intermediate group of lugs
- LW: Side lugs
- A: Terminal appendix
- 10: System (first embodiment)
- 12: Support structure
- 14: Retaining structure
- 16: Cavity
- 18: Inner sheet
- 20: Outer sheet
- 21: Segment of the periphery of the inner and outer sheets
- 22: Cover flap
- 22a: Boundary between opaque portion and transparent portion
- 22b: Transparent portion
- 24: Self-adhesive portion
- 26: Bearing flap
- 27: Tab or gripping tongue
- 27a: Proximal portion
- 27b: Distal portion
- 28: Retaining surface
- 30: Holding straps
- 32: Stop means
- 110: System (second embodiment)
- 114: Retaining structure (second embodiment)
- 133: Elastomeric material
- 134: Casing
- 136: Tray
- 138: Reclosable lid
- 140: First passageway
- 142: Second passageway
- 210: System (third embodiment)
- 212: Support structure
- 244: Wearable band
- 245: Pins
- 246: Through-holes
- 310: System (fourth embodiment)
- 312: Support structure
- 348: Patch
- 350: Support layer
- 352: Adhesive layer
- 410: System (fifth embodiment)
- 414: Retaining structure
- 433: Elastomeric material

## Claims

1. Protection and accommodation system (110; 210; 310) in particular for a device (C) for delivering a treatment to a patient; said system comprising:
- a support structure (12; 212) arranged to be attached in a removable manner to the patient's body, and
- a retaining structure (114) associated with said support structure (12; 212) and comprising a deformable material (133), arranged to be deformed by a compression performed by an operator which pushes a fixing portion (W) of said delivery device (C) against said deformable material (133) and arranged for embedding and retaining said fixing portion (W) by means of one, at least partial, elastic return performed by said deformable material (133) after the compression performed by the operator, said retaining structure including a casing (134) which includes a tray (136) that receives said deformable material (133) and a reclosable lid (138) able to be coupled with said tray (136), **characterized in that** said tray (136) and said lid (138) comprise a receptacle with a raised circumferential edge, **in that** the casing has at least a couple of passageways (140; 142) made by laterally opposite parts of the casing (134) and arranged to be passed through by at least one portion (I, O) of said delivery device (C), said passageways being provided on the tray (136) and/or on the lid (138) and **in that** said deformable material (133) has adhesive properties.

2. System according to claim 1, wherein said deformable material is an elastomeric material (133) comprising a polymer.

3. System according to claim 1 or 2, wherein said deformable material (133) is a polymer included in the list consisting of silicone, inert polyurethane, a clay-based material, a wax-based material, a shape-memory material, and a polyester-based material.

4. System according to any of the preceding claims, wherein said lid (148) is hinged to said tray (136).

5. System according to any of the preceding claims in which said support structure (12) is arranged for assuming a cover arrangement, wherein it is superposed to - and covers - said retaining structure (114), and a release arrangement, wherein it allows to reach such retaining structure (114); said support structure (12) defining a cavity (16) arranged to accommodate at least one portion (I, O) of said delivery device (C).

6. System according to claim 5, wherein said support structure (12) includes an inner sheet (18) and an outer sheet (20) overlapping one on the other and connected so as to form a pocket located at the space between them.

7. System according to claim 6, wherein said support structure (12) includes a cover flap (22) which is oscillatable so as to cover said retaining structure (114) when said support structure (12) is in the cover arrangement, and so as to allow to reach said retaining structure (114) when said support structure (12) is the release arrangement.

8. System according to claim 7, wherein said support structure (12) comprises a bearing flap (26) on which said retaining structure (114) is supported and which is arranged to be covered by said cover flap (22) when said support structure (12) is in said cover arrangement.

9. System according to any of the preceding claims, wherein said support structure (212; 312) is a wearable band (244) or patch (348).

## Patentansprüche

1. Schutz- und Aufnahmesystem (110; 210; 310), insbesondere für eine Vorrichtung (C) zum Verabreichen einer Behandlung an einem Patienten, wobei das System aufweist:
- eine Trägerstruktur (12;212) die dafür geeignet ist, abnehmbar am Körper eines Patienten befestigt zu werden, und
- eine Haltestruktur (114), die der Trägerstruktur (12, 212), zugeordnet ist und ein verformbares Material (133) aufweist, das dafür vorgesehen ist, durch von einem Benutzer ausgeübten Druck verformt zu werden, der einen Befestigungsabschnitt (W) der Verabreichungsvorrichtung (C) gegen das verformbare Material (133) schiebt, und dafür vorgesehen ist, den Fixierabschnitt (W) mittels einer zumindest teilweise elastischen Rückstellung, die von dem verformbaren Material (133) nach dem Zusammendrücken durch den Benutzer ausgeführt wird, einzubetten und zu halten, wobei die Haltestruktur ein Gehäuse (134) aufweist, das ein Fach (136), welches das verformbare Material (133) aufnimmt, und einen verschließbaren Deckel (138), der an das Fach (136) gekoppelt werden kann, aufweist, **dadurch gekennzeichnet, dass** das Fach (136) und der Deckel (138) eine Aufnahme mit einem erhabenen Umfangsrand aufweisen, und dass das Gehäuse wenigstens ein Paar Durchgänge (140, 142) hat, die von sich lateral gegenüberliegenden Teilen des Gehäuses (134) gebildet und dafür vorgesehen sind, dass durch sie wenigstens ein Abschnitt (I, O) der Verabreichungsvorrichtung (C) hindurch verläuft, wobei die Durchgänge an dem Fach (136) und/oder dem Deckel (138) vorgesehen sind, und dass das verformbare Material (133) klebende Eigenschaften hat.

2. System nach Anspruch 1, wobei das verformbare Material ein elastomeres Material (133) ist, das ein Polymer enthält.

3. System nach Anspruch 1 oder 2, wobei das verformbare Material (133) ein Polymer ist, das in der Liste enthalten ist, die aus Silikon, Polyurethan, einem Material auf Basis von Lehm, einem Material auf Basis von Wachs, einem Formgedächtnismaterial und einem Material auf Basis von Polyester besteht.

4. System nach einem der vorstehenden Ansprüche, wobei der Deckel (148) an dem Fach (136) angelenkt ist.

5. System nach einem der vorstehenden Ansprüche, wobei die Trägerstruktur (12) dafür vorgesehen ist, einen Abdeckzustand einzunehmen, in dem sie die Haltestruktur (114) überdeckt und bedeckt, und einen Freigabezustand, in dem sie Zugriff auf die Haltestruktur (114) ermöglicht, wobei die Trägerstruktur (12) einen Hohlraum (16) bildet, der dafür vorgesehen ist, wenigstens einen Abschnitt (I, O) der Verabreichungsvorrichtung (C) aufzunehmen.

6. System nach Anspruch 5, wobei die Trägerstruktur (12) ein inneres Blatt (18) und ein äußeres Blatt (20) aufweist, die sich überlappen und miteinander verbunden sind, um in dem Raum zwischen sich eine Tasche zu bilden.

7. System nach Anspruch 6, wobei die Trägerstruktur (12) eine Abdeckklappe (22) aufweist, die hin und her beweglich ist, so dass sie Haltestruktur (14) abdeckt, wenn die Trägerstruktur (12) in dem Abdeckzustand ist, und so, dass sie einen Zugriff auf die Haltestruktur (114) ermöglicht, wenn die Trägerstruktur (12) in dem Freigabezustand ist.

8. System nach Anspruch 7, wobei die Trägerstruktur (12) eine Trageklappe (26) aufweist, auf der die Haltestruktur (114) getragen wird und die dafür vorgesehen ist, von der Abdeckklappe (22) bedeckt zu werden, wenn die Trägerstruktur (12) in dem Abdeckzustand ist.

9. System nach einem der vorstehenden Ansprüche, wobei die Trägerstruktur (12, 212) ein tragbares Band (244) oder Pflaster (348) ist.

## Revendications

1. Système de protection et de logement (110 ; 210 ; 310) particulièrement destiné à un dispositif (C) servant à administrer un traitement à un patient ; ledit système comprenant :
- une structure de support (12 ; 212) conçue pour être attachée de façon amovible au corps du patient, et
- une structure de maintien (114) associée à ladite structure de support (12 ; 212) et comprenant un matériau déformable (133) conçu pour se déformer lors de l'application d'une compression par un opérateur qui pousse une partie de fixation (W) dudit dispositif (C) d'administration contre ledit matériau déformable (133), et conçu pour intégrer et maintenir ladite partie de fixation (W) au moyen d'un rappel élastique, au moins partiel, assuré par ledit matériau déformable (133) après la compression appliquée par l'opérateur, ladite structure de maintien comprenant un boîtier (134) qui comprend un plateau (136) qui reçoit ledit matériau déformable (133) et un couvercle (138) pouvant être refermé pouvant être couplé audit plateau (136), **caractérisé en ce que** ledit plateau (136) et ledit couvercle (138) comprennent un réceptacle doté d'un bord circonférentiel érigé, **en ce que** le boîtier comporte au moins deux passages (140 ; 142) fournis par des parties latéralement opposées du boîtier (134) et conçus pour être traversés par au moins une partie (I, O) dudit dispositif (C) d'administration, lesdits passages étant ménagés sur le plateau (136) et/ou sur le couvercle (138), et **en ce que** ledit matériau déformable (133) possède des propriétés adhésives.

2. Système selon la revendication 1, dans lequel ledit matériau déformable est un matériau élastomère (133) comprenant un polymère.

3. Système selon la revendication 1 ou 2, dans lequel ledit matériau déformable (133) est un polymère compris dans la liste comprenant du silicone, un polyuréthane inerte, un matériau à base d'argile, un matériau à base de cire, un matériau à mémoire de forme et un matériau à base de polyester.

4. Système selon l'une quelconque des revendications précédentes, dans lequel ledit couvercle (148) est articulé par rapport audit plateau (136).

5. Système selon l'une quelconque des revendications précédentes, dans lequel ladite structure de support (12) est conçue de façon à prendre une disposition de recouvrement, dans laquelle elle est superposée à ladite structure de maintien (114) - et la recouvre - et une disposition de libération, dans laquelle elle permet d'atteindre ladite structure de maintien (114) ; ladite structure de support (12) définissant une cavité (16) conçue pour loger au moins une partie (I, O) dudit dispositif (C) d'administration.

6. Système selon la revendication 5, dans lequel ladite structure de support (12) comprend une feuille intérieure (18) et une feuille extérieure (20) se chevauchant et reliées de façon à former une poche située au niveau de l'espace les séparant.

7. Système selon la revendication 6, dans lequel ladite structure de support (12) comprend un rabat de recouvrement (22) qui peut osciller de façon à recouvrir ladite structure de maintien (114) lorsque ladite structure de support (12) est dans la disposition de recouvrement, et de façon à pouvoir atteindre ladite structure de maintien (114) lorsque ladite structure de support (12) est la disposition de libération.

8. Système selon la revendication 7, dans lequel ladite structure de support (12) comprend un rabat porteur (26) sur lequel est supportée ladite structure de maintien (114) et qui est conçu pour être recouvert par ledit rabat de recouvrement (22) lorsque ladite structure de support (12) est dans ladite disposition de recouvrement.

9. Système selon l'une quelconque des revendications précédentes, dans lequel ladite structure de support (212 ; 312) est une bande (244) ou un timbre (348) pouvant être porté.
